# EUROPEAN PATENT SPECIFICATION

(11) **EP 1 531 872 B1**
(45) Date of publication and mention of the grant of the patent: **28.02.2007**
(21) Application number: 03790943.9
(22) Date of filing: 29.08.2003
(51) Int. Cl.: A61K 49/06

(54) **METHOD AND APPARATUS FOR PRODUCING CONTRAST AGENTS FOR MAGNETIC RESONANCE IMAGING**
VERFAHREN UND VORRICHTUNG ZUR HERSTELLUNG VON KONTRASTMITTELN FÜR MAGNETRESONANZBILDGEBUNG
PROCEDE ET DISPOSITIF DE PRODUCTION D'AGENTS DE CONTRASTE POUR L'IMAGERIE PAR RESONANCE MAGNETIQUE

(30) Priority: 29.08.2002 GB 0219952
(43) Date of publication of application: 25.05.2005
(73) Proprietor: GE Healthcare AS, 0485 Oslo (NO)
(72) Inventor: ARDENKJAER-LARSEN, Jan, Henrik, N-0401 Oslo (NO); AXELSSON, Oskar, N-0401 Oslo (NO); JOHANESSON, Haukur, N-0401 Oslo (NO)
(74) Representative: Franks, Barry Gerard
(86) International application number: PCT/EP2003/009590
(87) International publication number: WO 2004/019997

(56) References cited:
- WO-A-00/71166
- WO-A-02/23210
- GOLMAN K ET AL: "Parahydrogen-induced polarization in imaging: subsecond /sup 13/C angiography" MAGNETIC RESONANCE IN MEDICINE, JULY 2001, WILEY, USA, vol. 46, no. 1, pages 1-5, XP002263068 ISSN: 0740-3194
- BARKEMEYER J ET AL: "Heteronuclear polarization transfer using selective pulses during hydrogenation with parahydrogen" JOURNAL OF MAGNETIC RESONANCE, SERIES A, MAY 1996, ACADEMIC PRESS, USA, vol. 120, no. 1, pages 129-132, XP002263069 ISSN: 1064-1858

## Description

The present invention relates to an apparatus and method for para-hydrogen induced hyperpolarization of a compound, and particularly for the preparation of a contrast agent for magnetic resonance imaging procedures.

### Background of the Invention

Magnetic Resonance Imaging (MRI) is an important diagnostic technique. It is especially attractive since it is non-invasive and does not expose the patient to potentially harmful radiation such as X-rays or radiation from radioactive materials. Significant progress has recently been made in the quality of images as well as finding new applications of the technique. The progress relies on a rapid development of the digital image processing, the refined Nuclear Magnetic Resonance (NMR) techniques and the development of effective contrast agents (imaging agents). An emerging technique of particular interest involves Magnetic Resonance (MR) contrast agents based on the principle of pre-polarization of the nuclear spins, also called hyperpolarization.

For the resonance phenomena, which are the basis of NMR and MRI, to occur, isotopes with non-zero nuclear spin have to be present. In addition, since NMR is not an extremely sensitive technique, a relatively high concentration and/or a high gyromagnetic ratio is needed, especially for imaging purposes. The use of a selected isotope in a contrast agent which is to be injected in a patient, puts further requirements on the selected isotope, for example regarding toxicity. A number of isotopes have the required spin properties, but only a few are considered interesting for the use in contrast agents, for example the carbon isotope ¹³C and the nitrogen isotope ¹⁵N. The carbon isotope ¹³C has many properties that would it make it useful as a functional part of an MRI contrast agent. An important feature is the long longitudinal relaxation time, *T₁.* The relaxation time needs to be long in order to have time, after the generation of the contrast agent, to inject the contrast agent into the patient and to allow the contrast agent to be transported to the organ that is to be studied. To make a useful MR-contrast agent of this kind, the signal strength has to be boosted significantly over the thermal equilibrium signal. Patent application WO 00/71166, by the same applicant, describes a process and an apparatus for increasing the polarization and hence the signal from a small organic molecule containing for example ¹³C. The signal was increased with a factor 10⁴. The process is referred to as Para-Hydrogen Induced Polarisation (PHIP) and may comprise the transferring of nuclear spin-order from para-hydrogen to spin polarization in non-zero spin nuclei in molecules, e.g. to ¹³C or ¹⁵N nuclei.

Hydrogen molecules exist in four different spin states. In one of the forms, characterised by antiparallel spins, the magnetic moments of the protons cancel. This form is called para-hydrogen. The other three forms, with a net magnetic moment, are referred to as ortho-hydrogen. The para-hydrogen will not rotate at low temperature whereas the ortho-form must rotate with a high frequency at all temperatures because of quantum-mechanical symmetry requirements of the wave function. This indicates that at low temperature the para-form will have a significantly lower energy, and hence is the energetically favoured form. At temperatures below 20 K the equilibrium ratio of para- and ortho-hydrogen approaches 100:0, at 80 K the ratio is 48:52 and at room temperature approximately 1:3. The equilibration can be speeded up by the presence of a transition metal catalyst, e.g. Fe₂O₃. Para-hydrogen relaxes slowly (if no catalyst is present) at room temperature.

In WO 00/71166 it is described how to catalytically hydrogenate (with para-hydrogen) unsaturated compounds comprising non-zero spin nuclei such as ¹³C. The spin correlation of the protons from the para-hydrogen will be preserved during and after hydrogenation, and the influence on the spins of the ¹³C nuclei breaks the symmetry of the spin system. The protons will now give an NMR-signal, but the non-equilibrium spin order is not sufficient to make the molecule useful for imaging purposes since it has an anti-phase behaviour that is not ideal for imaging. In the above cited applications, and further in "Parahydrogen-Induced Polarization in Imaging" by K. Golman et al., Magnetic Resonance in Medicine 46:1-5 (2001), a magnetic field cycling method is described for transforming the proton spin-order to carbon spin polarization. In a first step the external magnetic field is reduced (from the relatively high geomagnetic field) bringing the combined proton-carbon spin system into its strong coupling regime. In this regime the scalar coupling (J-coupling) strongly influences the evolution of the spin system. The reduction of the field should be fast, giving a diabatic (non-adiabatic) process. In a subsequent step the field strength is slowly increased (an adiabatic process). The field cycling will result in a substantial increase in the polarization of the spins of the ¹³C nuclei, giving an in-phase NMR-signal, and increase the usefulness of the compound as a contrast agent for use in imaging procedures. However, the result of the field treatment will be dependent on the scalar coupling of the combined spin system and the properties of the magnetic field. In WO 00/71166 examples of field cycling schemes are described that give a substantial improvement in the image quality. To make the method even more attractive for use in medical and diagnostic applications it would be of high value to further increase the degree of polarization of the carbon spins and shorten the production times for PHIP contrast agents. At the same time the method should be easy to implement and not substantially increase the cost of the equipment for producing the contrast agent.

### Summary of the Invention

The object of the present invention is to provide a method and an apparatus for producing MRI contrast agent with a high degree of polarization of the imaging nuclei spins.

The object is achieved by the method as defined in claim 1, the apparatus as defined in claim 8, and by the computer program product as defined in claims 6 and 7.

The method for producing contrast agent according to the present invention comprises the steps of obtaining a solution in a solvent of a hydrogenatable, unsaturated substrate compound and a catalyst for the hydrogenation of a substrate compound, hydrogenating the substrate with hydrogen gas (H₂) enriched in para-hydrogen (p-¹H₂) to form a hydrogenated contrast agent and exposing the contrast agent to a sequence of pulses of magnetic field for enabling spin-order to be transferred from protons in the hydrogenated contrast agent to polarization of a nucleus within the same molecule for enhancing the contrasting effects of the contrast agent adapted for use in an MR application. Further, the dose or part of a dose of the contrast agent is exposed to an initial field followed by a first pulse of magnetic field having a first magnetic field strength, a first orientation and a first duration, and to one or more further subsequent pulses of magnetic field, wherein two subsequent pulses differ in at least one of the parameters: magnetic field strength, orientation or duration. After the last pulse in the sequence a magnetic field of the same order of magnetic field strength and direction as said initial field are applied to the contrast agent.

The apparatus for producing MR contrast agent according to the present invention comprises means for producing pulses of magnetic field, wherein the means for producing pulses of magnetic field comprises orthogonal Helmholtz pairs. The magnetic treatment unit may further comprises means for detecting the induced magnetic signal of the contrast agent, for example a number of pick-up coils arranged in different directions.

One advantage afforded by the apparatus and method according to the present invention is that contrast agent can be produced that significantly improves the image quality and/or speeds up the process in an MRI application and/or improves the analysis performance in an MNR application.

A further advantage is that novel types of imaging, not possible, or very difficult to perform with prior art techniques, can be performed.

The advantages are achieved by the apparatus and method according to the present invention by providing a high degree of polarization of the imaging nuclei of the contrast agent and by that the process of producing contrast agent is performed rapidly.

### Brief Description of the Drawings

The invention will now be described in detail with reference to the drawing figures, in which'
Fig. 1 is a schematic drawing illustrating the apparatus according to the invention;
Fig. 2 is a schematic drawing illustrating the magnetic treatment unit of the apparatus according to the invention;
Fig. 3 is a flowchart illustrating one embodiment of the method according to the invention;

### Detailed Description of the invention

Parts of apparatus and parts of the process described in WO 00/71166 are advantageously utilised also in the present invention. WO 00/71166 teaches that the hydrogenation reaction is preferably performed by mixing gaseous pare-hydrogen (or ortho-deuterium) enriched hydrogen with a solution of an unsaturated compound and a hydrogenation catalyst.

The present invention comprises a process having the following main steps
- 100:: obtaining a solution comprising a solvent, a hydrogenatable, unsaturated substrate compound and a catalyst for the hydrogenation of a substrate compound;
- 110:: introducing the solution into a chamber containing hydrogen gas (H₂) enriched in para-hydrogen (p-¹H₂) in order to hydrogenate the substrate to form a hydrogenated contrast agent;

The method according to the present invention introduces a main step of:
- 120:: subjecting the contrast agent to a sequence of magnetic field pulses.

The field pulses in the sequence are typically in the order of 1 mT, their duration in thee order of 10 ms. Typically the magnetic field pulses are applied in three orthogonal directions. The magnetic field pulse sequence enables spin-order to be transferred from protons in the freshly hydrogenated contrast agent into polarization of a nucleus within the same molecule with a slower relaxation, preferably a ¹³C or ¹⁵N nucleus. An apparatus and a method according to the present invention for providing a magnetic field pulse sequence during the production of the imaging agent is described below.

The hydrogenatable substrate used may be a material such as a para-hydrogenation substrate as discussed in WO99/24080. For *in vitro* or *in vivo* MR studies of biological or quasi-biological processes or synthetic polymer (e.g. peptide, polynucleic acid etc.) syntheses, the substrate is preferably hydrogenatable to form a molecule participating in such reactions, e.g. an amino acid, a nucleic acid, a receptor-binding molecule, etc., either a natural such molecule or an analog.

The solvent used in step 100 of the process of the invention may be any convenient material which serves as a solvent for the substrate and the hydrogenation catalyst. A number of possible solvents are discussed in WO99/24080. When the contrast agent is for use in *in vivo* MR investigations, the solvent is preferably physiologically tolerable. Water is a preferred choice of solvent, used in combination with a water-soluble catalyst. If other solvents that are not physiologically tolerable are used, the solvents has to be removed before use in a patient, for example by vacuum-spray. Other rapid solvent removal techniques, e.g. affinity techniques, may however be used. The solvent is preferably used at or near the minimum quantities required to maintain substrate, catalyst and contrast agent in solution without experiencing viscosity problems during the hydrogenation reaction.

The hydrogenation catalyst is preferably a catalyst as discussed in WO99/24080, e.g. a metal complex, in particular a rhodium complex.

The enriched hydrogen, which may be pure ¹H₂ or ²H₂, or a mixture of ¹H₂ and ²H₂, may optionally contain other gases, but is preferably free from oxygen or other reactive or paramagnetic gases, may be prepared by cooling hydrogen, preferably to a temperature below 80 K, more preferably to below 50 K, even more preferably to below 30 K and especially preferably to below 22 K, and allowing the nuclear spin states to equilibrate, optionally in the presence of a solid phase equilibration promoter, e.g. Fe₃O₄, Fe₂O₃, activated charcoal, etc. The enriched hydrogen is then preferably removed from the equilibrator and optionally stored before use. A method of preparation and storage of enriched hydrogen is described in WO99/24080. A preferred and novel method of storage and equipment for that purpose has been developed by the inventors. The enriched hydrogen is transferred to and stored in gas cylinders made of inert material. Inert in this context should be understood as made up by material essentially free from paramagnetic materials (primarily iron) and other para-hydrogen relaxing compounds (e.g. palladium). Examples of inert materials suitable for the gas cylinders are aluminum and carbon fiber reinforced epoxy. The enriched hydrogen will decay slowly (in the order of 10% per week). Such a decay rate is acceptable in most applications, especially compared with the cost and handling problems of previous storing methods involving storing at cryogenic temperatures.

For the hydrogenation reaction, a reaction chamber is filled with enriched hydrogen optionally under pressure, preferably 5-20 bar, and the catalyst and substrate solution is introduced either as a thin jet, by spraying or by atomising, into this reactor. If desired, the solution may be produced by mixing separate solutions of catalyst and of substrate. To ensure proper mixing, a distributor or a plurality of spray nozzles may be used and the chamber contents may be mixed, e.g. by a mechanical stirrer or by appropriately shaping the chamber walls to promote turbulent mixing when there is a flow of reaction mixture in the chamber.

The process may be performed continuously with a flow reactor, e.g. a loop or tube reactor, or alternatively it may be a batch-wise process. Preferably there will be a continuous or pulsed flow of enriched hydrogen and solution into the reactor, a continuous or batch-wise removal of liquid solution from the base of the reactor, and a continuous or batch-wise venting of unreacted gas from the reactor. The enriched hydrogen and solution passing into the reactor are preferably temperature-controlled to ensure the gas/droplet phase in the reactor is at the desired temperature. This can be achieved by providing input lines with temperature sensors and heating and/or cooling jackets.

If a non-aqueous solution has been used the contrast agent is preferably mixed with water after the hydrogenation and the low magnetic field treatment. The water used is preferably sterile and also preferably essentially free of paramagnetic contaminants. The resultant aqueous solution is then preferably treated to remove the hydrogenation catalyst, e.g. by passage through an ion exchange column, preferably one free of paramagnetic contaminants. The water may be temperature-controlled as may be a mixing chamber where water and contrast agent solutions are mixed so as to ensure the aqueous solution enters the ion exchange column at the appropriate temperature. Strongly acidic, sodium ion charged ion exchange resins such as DOWEX 1x2-400 (Dow Chemicals) and Amberlite IR-120 (both available from Aldrich Chemicals) resins may conveniently be used for the removal of typical metal complex hydrogenation catalysts. For fast ion exchange, the resin is preferably cross-linked to only a low degree, e.g. a 2% divinyl benzene cross-linked sulphonated, sodium ion loaded polystyrene resin.

Removal of the non-aqueous solvent may then conveniently be effected by spray flash distillation e.g. by spraying the aqueous solution into a chamber, applying a vacuum, and driving the organic solvent free aqueous solution from the chamber using an inert, preferably non-paramagnetic gas, e.g. nitrogen. Indeed in general the flow of liquid components through the hydrogenation apparatus is preferably effected using applied nitrogen pressure, e.g. 2 to 10 bar.

The resulting aqueous contrast agent solution may be frozen and stored or may preferably be used directly in an MR imaging or spectroscopy procedure, optionally after dilution or addition of further solution components, e.g. pH modifiers, complexing agents, etc. Such direct use may for example involve continuous infusion or alternatively injection or infusion of one or more dose units. Bolus injection is particularly interesting.

The whole process from beginning of hydrogenation to the delivery of the finished contrast agent in for example a syringe may conveniently be effected in less than 100 seconds, indeed i it is feasible to produce dosage units in less than 10 seconds, which is substantially less than T₁ for the potentially interesting imaging nuclei.

Preferably, the surfaces contacted by the contrast agent during the process of the invention are substantially free of paramagnetic materials, e.g. made of glasses as used for hyperpolarized ³He containment as discussed in WO99/17304 or gold or polymer, optionally deuterated. Surfaces contacting a non-aqueous solvent (e.g. acetone) should be acetone-resistant and; valves may be magnetically controlled and provided with solvent resistant Teflon or silicone parts.

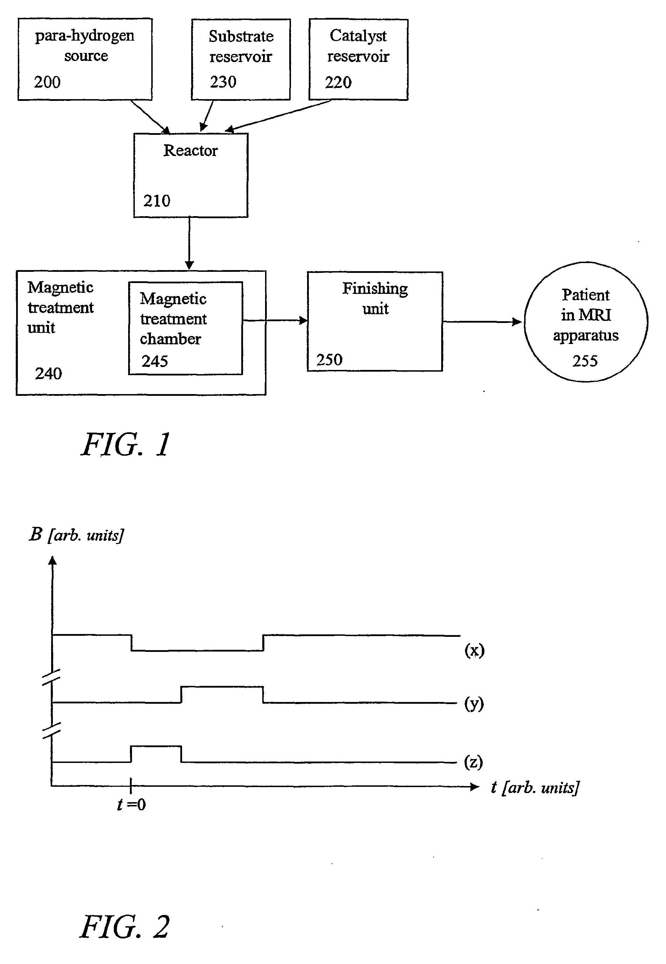

An apparatus suitable for producing contrast agent using the method according to the present invention will be described with reference to figure 1. Hydrogen (¹H₂) enriched in para-hydrogen is fed from the para- hydrogen source 200 into a reactor 210. A hydrogenation catalyst solution from a catalyst reservoir 230 and a hydrogenatable substrate solution from a substrate reservoir 220 are fed into the reactor 210. The liquid settling in reactor 210 is transferred to a magnetic treatment unit 240, which essentially comprises a magnetic treatment chamber 245 for receiving a dosage of the contrast agent surrounded by means for producing the magnetic pulses, and thence to finishing unit 250 for cleanup, quality control and possible addition of additives and solvent removal. The finishing unit may comprise an ion exchange column and a solvent removal chamber equipped with a spray nozzle. After the passage through the finishing units the contrast agent is delivered to, for example, a syringe for injection in a patient. Alternatively the contrast agent is stored for later usage. Alternatively, the magnetic treatment may be performed directly in the reactor 210, which then would be equipped with means for producing the magnetic pulses. Thus the need for a separate magnetic treatment chamber 245 is eliminated.

The magnetic treatment unit 240 comprises orthogonal Helmholtz pairs. The magnitude of the produced magnetic field and its orientation is determined by the currents flowing in the coils. The currents are typically provided by high precision computer controlled current supplies (not shown). As will be discussed below, the field amplitude and/or the duration of each pulses period are critical for the result. To achieve a high accuracy the field amplitude can be controlled electronically, for example with flux-gate stabilization, or the length of each period can be made dynamic if the induced signals are detected. Hence the magnetic treatment unit 240 is preferably provided with means for detecting the induced signal, for example pick-up coils in the x, y and z-directions. Signal detection also provides a means for measuring the polarization degree, which is useful for checking the quality of the produced contrast agent, and especially in the process of developing, and/or fine-tuning, a pulse sequence.

Preferably, most of the functions are integrated in a PC environment, in particular the generation and control of the magnetic field pulse sequence.

In the method of the present invention the contrast agent is exposed to a series of sudden field changes. The field changes are characterized by sudden, step-like increases and decreases. The principles of a magnetic field pulse sequence according to the method of the invention will be described with reference to figure 2. The contrast agent is initially exposed to a initial constant field in an initial direction (x), the initial field is typically less than 1 mT, for example the earth magnetic field. This initial field is reduced to zero in step-like fashion. At essentially the same time a field is applied (step-like) in a first direction (z), different from the initial direction. This constitutes the start of the field pulse sequence. The field in the first direction (z) is maintained for a time-period, *t₁,* in the order of 1-100 ms, and the amplitude of the field is typically in the order of 1 mT. The field in the first direction (z) is removed analogous to the step-like increase. The step-like increase, the short period, *t₁,* of constant field and the step-like decrease forming the first magnetic field pulse of the magnetic field pulse sequence. At essentially the time of the removal of the field in the first direction (z), a field in a second direction (y) is applied (step-like), maintained for a time period, *t₂,* and removed in a step-like fashion, forming a second field pulse. Subsequently a similar field pulse is applied in a third direction (x), typically the same direction as the initial direction, the duration and amplitude of which is in the same order as the previous fields. If this is the last pulse in the sequence, the magnetic field should typically be maintained at a constant level, preferably at the same level as the initial field as long as the dose remains in the magnetic field treatment chamber. The order of the pulses, their amplitudes and durations here described, should be considered as an example and not limiting to the scope of the invention.

The fields is generated within the magnetic treatment chamber with the use of orthogonal Helmholtz pairs. The current flowing in the coils will determine the magnitude and orientation.

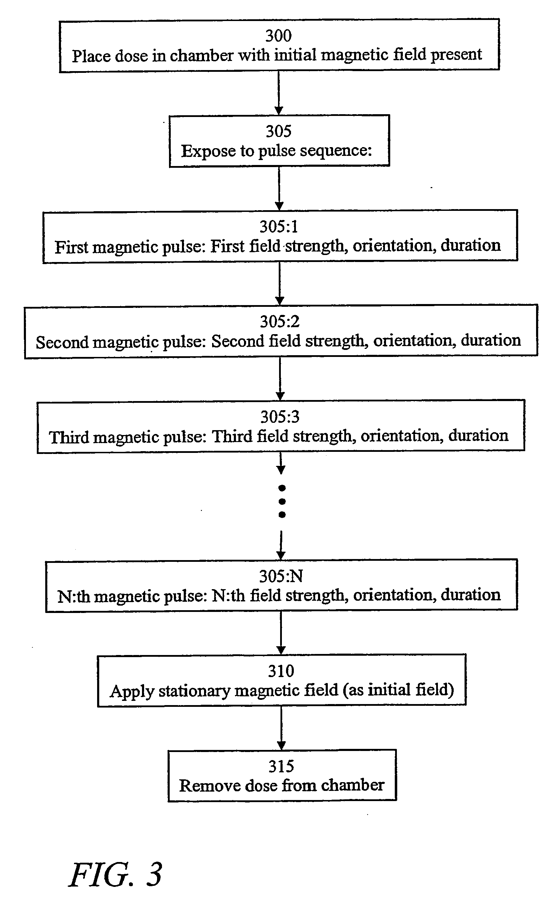

The method according to the present invention of low magnetic field treatment is illustrated in the flowchart of figure 3. The method comprises the steps of:
- 300:: The dose or part of the dose of contrast agent is placed in the magnetic field treatment chamber 245. A initial magnetic field in the order of the earth magnetic field should be present in the chamber when the sample is placed therein.
- 305:: The contrast agent is exposed to a magnetic field pulse sequence, comprising the sub-steps of:
- 305:1: The contrast agent is exposed to a first short magnetic pulse having a first field strength, a first orientation and a first duration.
- 305:2: The contrast agent is exposed to a second short magnetic pulse having a second field strength, a second orientation and a second duration.
- 305:3: The contrast agent is exposed to a third short magnetic pulse having a third field strength, a third orientation and a third duration. . . .
- 305:n: The contrast agent is exposed to a n:th short magnetic pulse having a lath field strength, a n:th orientation and a n:th duration. . . .
- 305:N: The contrast agent is exposed to a N:th short magnetic pulse having N:th field strength, a N:th orientation and a N:th duration.
- 310:: A magnetic field preferably of the same order and direction as the initial field of step 300 is applied.
- 315:: The dose, or part of the dose of contrast agent is removed from the low magnetic field treatment chamber and transferred to the remaining (chemical) process steps performed prior to injection of the contrast agent.

Where step 305:1 correspond to a first pulse, step 305:2 to a second, 305:n to a n:th and step 305:N to the N:th pulse. N is the total number of pulses.

The field amplitude, the duration of each pulse period and the number of pulses (N) are critical for the result. Analysis and simulation of the spin system give indications on how to design the field pulse series. A suitable sequence for the used compound can, if the compound is relatively simple, be calculated or simulated/optimized from quantum mechanical considerations. Sequences suitable for more complex compounds are typically established from experiments. Also sequences for simpler compounds can typically be improved by being fine-tuned by experiments. The field amplitude can be controlled either electronically (flux-gate stabilization) or the length of each period can be made dynamic if the induced signals are detected. Signal detection also provides a means for measuring the polarization degree.

The dynamic control comprises measuring the rotations of the magnetization vector by the current induced in the pick-up coils. Hence, the number of revolutions can be counted and the field may be switched at exactly the right moment. The dynamic control obviates the need for extremely high precision, high speed control of the magnetic field.

The embodiment of the present invention utilizing field pulses will be illustrated with the following examples: The hydrogenated molecule in the examples is maleic acid, the spin system which is of AAX type. The J-couplings are 15.5 Hz, 10.65 Hz, and 0.3 Hz. The sample is exposed to the field pulse series according to table 1:

| Time | Field |
|---|---|
| t≤0, | B=(0,0,1.0mT) |
| t∈]0;8.1ms], | B=(1.0mT,0,0) |
| t∈]8.1ms;28.0ms], | B=(0,0,1mT) |
| t∈]28.0;61.0ms], | B=(1.0mT,0,0) |
| t>=61.0ms, | B=(0,0,1.0mT) |

| | |
|---|---|
| *Table 1: Example of a field pulse series.* | |

The notations in table 1 should be understood as follows: t∈]8.1ms;28.0ms] indicates a pulse starting at time t=8.1.ms and ending at t=28.0 ms, i.e. the pulse has a duration of 19.9 ms; B=(0,0,1.0mT) indicates a magnetic field of strength 1.0mT in the z-direction of a laboratory frame of reference defined as the direction of the field in the surrounding of the equipment and the coordinate system is right-handed orthonormal. B=(1.0mT,0,0) indicates a pulse of the same field strength but directed along the x-axis.

At the end of this sequence the polarization of the carbon nuclei is -54%. Increasing the time of the last period by 0.140ms will change the sign of the polarization. The polarization oscillates with this period for the given example, and thus the field amplitude is critical for the sequence timing.

Another pulsed field example using the same spin system is shown in table 2 (notations as before):

| Time | Field |
|---|---|
| t≤0, | B=(0,0,0.1mT) |
| t∈]0;36.405ms], | B=(0.1mT,0,0) |
| t∈]36.405ms;85.905ms(49.500ms)], | B=(0,0,0.1mT) |
| t∈]85.905ms; 116.720ms(30.815ms)], | B=(0.1mT,0,0) |
| t>=116.720ms, | B=(0,0,0.1mT) |

| | |
|---|---|
| *Table* 2: *Example of a field pulse series.* | |

At the end of this sequence the polarization of the carbon is -89%. The examples clearly demonstrate the dependence of the net polarization on both the amplitude and the duration of the field pulses.

The method is preferably implemented by means of a computer program product comprising the software code means for performing the steps of the method by controlling parts of the apparatus according to the invention. The computer program product is typically executed on the computer controlling the apparatus. The computer program is loaded directly or from a computer usable medium, such as a floppy disc, a CD, the Internet etc.

From the invention thus described, it will be obvious that the invention may be varied in many ways. Such variations are not to be regarded as a departure from the inventive concept, and all such modifications as would be obvious to one skilled in the art are intended for inclusion within the scope of the following claims.

## Claims

1. A method for producing of MR contrast agent, the method comprising the steps of:
- obtaining a solution in a solvent of a hydrogenatable, unsaturated substrate compound and a catalyst for the hydrogenation of a stubstrate compound, wherein the substrate compound comprises imaging nuclei;
- hydrogenating the substrate with hydrogen gas (H₂) enriched in para-hydrogen (p-¹H₂) to form a hydrogenated contrast agent;
- exposing the contrast agent to a sequence of pulses of magnetic field for enabling spin-order to be transferred form protons in the hydrogenated contrast agent to polarization of a nucleus within the same molecule for enhancing the contrasting effects of the contrast agent adapted for use in MR application, wherein the exposing step comprises the steps of:
- placing (300) a dose or part of a dose of the contrast agent in a magnetic field treatment chamber (245) having a magnetic field in the order of the earth magnetic field;
- subjecting (305:1-305:N) the dose or part of a dose of the contrast agent to a first pulse of magnetic field having a first magnetic field strength, a first orientation and a first duration, and to one or more further subsequent pulses of magnetic field, wherein two subsequent pulses differ in orientation and optionally at least one of the parameters: magnetic field strength or duration;
- applying (310) to the dose or part of a dose of the contrast agent a magnetic field of the same order of magnetic field strength and direction as said initial field.

2. The method of claim 1 wherein the pulses of magnetic field are realized through the steps of :
- rapidly increasing the magnetic in one orientation;
- maintaining the magnetic field at a constant level and orientation for a predetermined duration;
- rapidly decreasing the magnetic field.

3. The method according to claim 1 wherein the subsequent pulses of magnetic field follow essentially immediately after each other.

4. The method according to claim 2 wherein the magnetic field is increased from an essentially zero-field to a magnetic field with a field strength in the interval of 0.1-1 mT.

5. The method according to claim 2 wherein the duration of the contrast magnetic field is in the interval of 1-100 ms.

6. A computer program product directly loadable into the internal memory of a processing means within a processing unit for controlling the method and apparatus for producing MR contrast agent, comprising the software code means adapted for controlling the steps of any of the claims 1 to 5.

7. A computer program stored on a computer readable medium, comprising a readable program adapted for causing a processing means, in a processing unit for controlling the method and apparatus for producing MR contrast agent, to control an execution of the steps of any of the claims 1 to 5.

8. Apparatus for producing MR contrast agent, the apparatus comprising a magnetic treatment unit (240) adapted for magnetic treatment of the contrast agent, **characterised in that** the magnetic treatment unit (240) comprises means for producing pulses of magnetic field wherein said means for producing pulses of magnetic field comprises orthogonal Helmholtz pairs.

9. Apparatus according to claim 8 wherein the magnetic treatment unit (240) further comprises means for detecting the induced magnetic signal of the contrast agent.

10. Apparatus according to claim 9 wherein the means for detecting the induced magnetic signal comprises pick-up coils in more than one direction.

## Patentansprüche

1. Ein Verfahren zum Herstellen eines MR-Kontrastmittels, wobei das Verfahren die Schritte umfasst:
- In einem Lösungsmittel Gewinnen einer Lösung einer hydrierbaren, ungesättigten Substratverbindung und eines Katalysators für die Hydrierung einer Substratverbindung, wobei die Substratverbindung Bildgebungskerne umfasst;
- Hydrieren des Substrats mit Wasserstoffgas (H₂), das in para-Wasserstoff (p-¹H₂) angereichert ist, um ein hydriertes Kontrastmittel zu bilden;
- Aussetzen des Kontrastmittel einer Sequenz von Pulsen eines Magnetfeldes, zum Ermöglichen, dass eine Spinordnung von Protonen im hydrierten Kontrastmittel zur Polarisation eines Kernes innerhalb desselben Moleküls übertragen wird, zum Verstärken der Kontrastierungseffekte des Kontrastmittels, das zur Verwendung in einer MR-Anwendung angepasst ist, wobei der Schritt des Aussetzens die Schritte umfasst:
- Anordnen (300) einer Dosis oder Teile einer Dosis des Kontrastmittels in einer Magnetfeldbehandlungskammer (245) mit einem Magnetfeld in der Größenordnung des Erdmagnetfeldes;
- Unterziehen (305:1-305:N) der Dosis oder des Teils einer Dosis des Kontrastmittels einem ersten Puls eines Magnetfeldes mit einer ersten Magnetfeldstärke, einer ersten Orientierung und einer ersten Dauer, und einem oder mehreren weiteren nachfolgenden Pulsen eines Magnetfeldes, wobei sich zwei nachfolgende Pulse in der Orientierung und gegebenenfalls mindestens einem der Parameter unterscheiden: Magnetfeldstärke oder Dauer;
- Anlegen (310) eines Magnetfeldes derselben Größenordnung von Magnetfeldstärke und Richtung wie das ursprüngliche Feld an die Dosis oder den Teil einer Dosis des Kontrastmittels.

2. Verfahren nach Anspruch 1, wobei die Pulse eines Magnetfeldes realisiert werden durch die Schritte:
- schnelles Vergrößern des Magnetischen in einer Richtung;
- Halten des Magnetfeldes auf einem/einer konstanten Niveau und Orientierung für eine vorbestimmte Dauer;
- schnelles Verkleinern des Magnetfeldes.

3. Verfahren nach Anspruch 1, bei dem die nachfolgenden Pulse eines Magnetfeldes im wesentlichen unmittelbar nacheinander folgen.

4. Verfahren nach Anspruch 2, bei dem das Magnetfeld vergrößert wird von einem im wesentlichen Null-Feld zu einem Magnetfeld mit einer Feldstärke im Intervall von 0,1 bis 1 mT.

5. Verfahren nach Anspruch 2, bei dem die Dauer des Kontrastmagnetfeldes im Intervall von 1 bis 100 ms liegt.

6. Computerprogrammprodukt, das direkt in den internen Speicher eines Verarbeitungsmittels innerhalb einer Verarbeitungseinheit zum Steuern des Verfahrens und der Vorrichtung zum Herstellen von MR-Kontrastmittel ladbar ist, umfassend das Softwarecode-Mittel, das zum Steuern der Schritte nach einem beliebigen der Ansprüche 1 bis 5 angepasst ist.

7. Computerprogramm, gespeichert auf einem Computer-lesbaren Medium, umfassend ein lesbares Programm, das angepasst ist zum Veranlassen eines Verarbeitungsmittels, in einer Verarbeitungseinheit zum Steuern des Verfahrens und der Vorrichtung zum Herstellen von MR-Konstrastmittel, zum Steuern eines Ausführens der Schritte nach einem der Ansprüche 1 bis 5.

8. Vorrichtung zum Herstellen von MR-Kontrastmittel, wobei die Vorrichtung umfasst eine Einheit (240) einer magnetischen Behandlung, angepasst zur magnetischen Behandlung des Kontrastmittels, **dadurch gekennzeichnet, dass** die Einheit (240) einer magnetischen Behandlung ein Mittel zum Herstellen von Pulsen eines Magnetfeldes umfasst, wobei das Mittel zum Herstellen von Pulsen eines Magnetfeldes orthogonale Helmholtz-Paare umfasst.

9. Vorrichtung nach Anspruch 8, bei der die Einheit (240) einer magnetischen Behandlung weiter umfasst ein Mittel zum Detektieren des induzierten magnetischen Signals des Kontrastmittels.

10. Vorrichtung nach Anspruch 9, bei der das Mittel zum Detektieren des induzierten magnetischen Signals Aufnahmespulen in mehr als einer Richtung umfasst.

## Revendications

1. Procédé de production d'agent de contraste de RM, le procédé comprenant les étapes de :
- obtention d'une solution dans un solvant d'un composé de substrat insaturé pouvant être hydrogéné et d'un catalyseur d'hydrogénation d'un composé de substrat dans lequel le composé de substrat comprend des noyaux d'imagerie ;
- hydrogénation du substrat avec du gaz hydrogène (H₂) enrichi en parahydrogène (p-¹H₂) afin de former un agent de contraste hydrogéné ;
- exposition de l'agent de contraste à une séquence d'impulsions de champ magnétique afin de permettre le transfert d'ordre de spin à partir de protons dans l'agent de contraste hydrogéné en polarisation d'un noyau à l'intérieur de la même molécule afin d'améliorer les effets de contraste de l'agent de contraste adapté à une utilisation dans des applications de RM, dans lequel l'étape d'exposition comprend les étapes de :
- mise en place (300) d'une dose ou partie d'une dose de l'agent de contraste dans une chambre de traitement sous champ magnétique (245) présentant un champ magnétique de l'ordre du champ magnétique terrestre ;
- exposition (305 :1 à 305 :N) de la dose ou d'une partie de la dose de l'agent de contraste à une première impulsion de champ magnétique présentant une première intensité de champ magnétique, une première orientation et une première durée, et à une ou plusieurs autres impulsions de champ magnétique ultérieures, dans lequel deux impulsions ultérieures diffèrent en orientation et, en variante, sur au moins l'un des paramètres : l'intensité ou la durée de champ magnétique ;
- application (310) sur la dose ou une partie d'une dose de l'agent de contraste d'un champ magnétique d'une intensité et une direction de champ magnétique du même ordre que celles dudit champ initial.

2. Procédé selon la revendication 1, dans lequel les impulsions de champ magnétique sont réalisées au moyen des étapes consistant à :
- augmenter rapidement le champ magnétique suivant une orientation ;
- maintenir le champ magnétique à un niveau et une orientation constants pendant une durée prédéterminée ;
- diminuer rapidement le champ magnétique.

3. Procédé selon la revendication 1, dans lequel les impulsions de champ magnétique ultérieures se suivent sensiblement immédiatement l'une après l'autre.

4. Procédé selon la revendication 2, dans lequel le champ magnétique augmente d'un champ sensiblement nul à un champ magnétique présentant une intensité de champ dans la plage de 0,1 à 1 mT.

5. Procédé selon la revendication 2, dans lequel la durée du champ magnétique de contraste est dans la plage de 1 à 100 ms.

6. Produit formant programme informatique pouvant être chargé directement dans la mémoire interne d'un moyen de traitement à l'intérieur d'une unité de traitement afin de commander le procédé et le dispositif de production d'agent de contraste de RM, comprenant un moyen formant code logiciel adapté afin de commander les étapes selon l'une quelconque des revendications 1 à 5.

7. Programme informatique mémorisé sur un support pouvant être lu par ordinateur, comprenant un programme pouvant être lu, adapté afin d'amener un moyen de traitement, dans une unité de traitement destinée à commander le procédé et le dispositif de production d'agent de contraste de RM, à commander l'exécution des étapes selon l'une quelconque des revendications 1 à 5.

8. Dispositif de production d'agent de contraste de RM, le dispositif comprenant une unité de traitement magnétique (240) adaptée afin d'assurer le traitement magnétique de l'agent de contraste, **caractérisé en ce que** l'unité de traitement magnétique (240) comprend un moyen destiné à produire des impulsions de champ magnétique, dans lequel ledit moyen de production d'impulsions de champ magnétique comprend des paires de Helmholtz orthogonales.

9. Dispositif selon la revendication 8, dans lequel l'unité de traitement magnétique (240) comprend, en outre, un moyen de détection du signal magnétique induit de l'agent de contraste.

10. Dispositif selon la revendication 9, dans lequel le moyen destiné à détecter le signal magnétique induit comprend des bobines d'acquisition suivant plus d'une direction.
